# EUROPEAN PATENT APPLICATION

(11) **EP 4 548 934 A1**
(43) Date of publication of application: **07.05.2025**
(21) Application number: 23831571.7
(22) Date of filing: 29.06.2023
(51) Int. Cl.: A61K 45/00, A61K 31/404, A61K 31/445, A61K 31/4468, A61K 31/472, A61K 31/5375, A61P 17/06

(54) **PHARMACEUTICAL COMPOSITION**

(30) Priority: 30.06.2022 JP 2022106025
(71) Applicant: Gexval Inc., Fujisawa-shi, Kanagawa 251-0012 (JP)
(72) Inventor: HIRASE, Ryo, Osaka-shi, Osaka 531-0071 (JP); ITO, Hiroaki, Osaka-shi, Osaka 531-0071 (JP); YASUNAGA, Yuka, Osaka-shi, Osaka 531-0071 (JP)
(74) Representative: Hoffmann Eitle
(86) International application number: PCT/JP2023/024149
(87) International publication number: WO 2024/005132

(57) **Abstract**

The present invention provides a novel therapeutic or prophylactic agent for psoriasis. This therapeutic or prophylactic agent for psoriasis contains a serotonin 4 receptor agonist.

## Description

### TECHNICAL FIELD

The present invention relates to a therapeutic or prophylactic agent for psoriasis.

### BACKGROUND ART

Psoriasis is a chronic inflammatory skin disease with a worldwide incidence of 2%. Psoriasis is characterized by inflammation of individual areas of the skin with scaling, redness, thickening, severe peeling, and, in some cases, itching, and can have considerable psychosocial effects on the quality of life and health of patients.

Psoriasis is primarily treated with topical and oral medications, biologics, and phototherapy.

Recently, oral medications for the treatment of psoriasis with PDE4 inhibitors (e.g., apremilast) as active ingredients have been developed.

Non-Patent Literature 1 describes that the transition of patients with mild psoriasis to systemic treatment was halved in those taking SSRIs (Selective Serotonin Reuptake Inhibitors).

However, the effectiveness of serotonin 4 receptor agonists in the treatment and prevention of psoriasis has not been conventionally known.

### Citation List

### Non-Patent Literature

Non-Patent Literature 1: Shirin ESKELAND et al., Acta Derm Venereol 2017; 97: 897-905

### SUMMARY OF INVENTION

### Technical Problem

It is an object of the present invention to provide a novel therapeutic or prophylactic agent for psoriasis.

### Solution to Problem

The inventors of the present invention conducted an in-depth study in order to address the above-described issues, and found that mosapride, which is widely used to improve gastrointestinal symptoms due to its gastroprokinetic action, is surprisingly effective for psoriasis. The inventors of the present invention conducted a further study, and accomplished the present invention on the basis of the finding that a group of compounds having the serotonin 4 receptor agonist action is effective in the treatment and prevention of psoriasis.

That is to say, the present invention is as follows.
[1] A therapeutic or prophylactic agent for psoriasis, containing a serotonin 4 receptor agonist.
[2] The agent according to [1], wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.
[3] The agent according to [1] or [2], which is for oral administration.
[4] A method for treating or preventing psoriasis, including a step of administering an effective amount of serotonin 4 receptor agonist to a subject.
[5] The method according to [4], wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.
[6] The method according to [4] or [5], wherein the administration is oral administration.
[7] A serotonin 4 receptor agonist for use in treatment or prevention of psoriasis.
[8] The serotonin 4 receptor agonist according to [7], wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.
[9] The serotonin 4 receptor agonist according to [7] or [8], for use in oral administration.
[10] Use of a serotonin 4 receptor agonist in the manufacture of a therapeutic or prophylactic agent for psoriasis.
[11] The use according to [10], wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.
[12] The use according to [10] or [11], wherein the therapeutic or prophylactic agent for psoriasis is for oral administration.
[13] A therapeutic or prophylactic agent for psoriasis, containing a serotonin 4 receptor agonist.
[14] The agent according to [13], wherein the serotonin 4 receptor agonist is one or at least two selected from the group consisting of mosapride, prucalopride, velusetrag, tegaserod, cisapride, cinitapride, clebopride, fenfluramine, felcisetrag, naronapride, usmarapride, relenopride, renzapride, CJ-033466, and pharmaceutically acceptable salts thereof (preferably one or at least two selected from the group consisting of mosapride, prucalopride, velusetrag, tegaserod, cisapride, cinitapride, clebopride, and pharmaceutically acceptable salts thereof).
[15] The agent according to [13] or [14], which is orally administered.

### Advantageous Effects of Invention

According to the present invention, it is possible to provide a novel therapeutic or prophylactic agent for psoriasis.

The therapeutic or prophylactic agent for psoriasis of the present invention is expected to have a remarkable effect in the treatment or prevention of psoriasis.

### Brief Description of Drawings

[FIG. 1] FIG. 1 shows results of Test Example 1. In the diagram, mg/kg indicates mg/kg body weight.
[FIG. 2] FIG. 2 shows results of Test Example 2. In the diagram, mg/kg indicates mg/kg body weight.
[FIG. 3] FIG. 3 shows results of Test Example 3. In the diagram, mg/kg indicates mg/kg body weight.
[FIG. 4] FIG. 4 shows results of Test Example 4. In the diagram, mg/kg indicates mg/kg body weight.
[FIG. 5] FIG. 5 shows results of Test Example 5. In the diagram, mg/kg indicates mg/kg body weight.
[FIG. 6] FIG. 6 shows results of Test Example 6. In the diagram, mg/kg indicates mg/kg body weight.
[FIG. 7] FIG. 7 shows results of Test Example 7. In the diagram, mg/kg indicates mg/kg body weight.

### DESCRIPTION OF EMBODIMENTS

The therapeutic or prophylactic agent for psoriasis of the present invention (which may be abbreviated as the "agent of the present invention" in this specification) contains a serotonin 4 receptor agonist as an active ingredient.

In the present invention, examples of the serotonin 4 receptor agonist include mosapride, prucalopride, velusetrag, tegaserod, cisapride, cinitapride, clebopride, fenfluramine, felcisetrag, naronapride, usmarapride, relenopride, renzapride, CJ-033466, revexepride, E-3620, and pharmaceutically acceptable salts thereof, preferably include mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof, and more preferably include mosapride, prucalopride, velusetrag, and pharmaceutically acceptable salts thereof. In the present invention, these serotonin 4 receptor agonists may be used alone or in a combination of two or more.

In the present invention, the concept of the serotonin 4 receptor agonist also encompasses solvates (e.g., hydrates), if solvates (e.g., hydrates) are present.

In the present invention, examples of the pharmaceutically acceptable salts of mosapride include mosapride citrate (in particular, mosapride citrate dihydrate), examples of the pharmaceutically acceptable salts of prucalopride include prucalopride succinate, examples of the pharmaceutically acceptable salts of tegaserod include tegaserod maleate, examples of the pharmaceutically acceptable salts of cinitapride include cinitapride tartrate, examples of the pharmaceutically acceptable salts of clebopride include clebopride malate, examples of the pharmaceutically acceptable salts of relenopride include relenopride hydrochloride, examples of the pharmaceutically acceptable salts of renzapride include renzapride hydrochloride (in particular, renzapride hydrochloride dihydrate), and examples of the pharmaceutically acceptable salts of naronapride include naronapride hydrochloride.

The therapeutic or prophylactic agent for psoriasis of the present invention may be administered orally or parenterally, but oral administration is preferred.

If the therapeutic or prophylactic agent for psoriasis of the present invention is provided as a pharmaceutical product for oral administration, examples of the dosage form thereof include tablets, capsules, powders, granules, lozenges, syrups, solutions, suspensions, emulsions, and the like.

If the therapeutic or prophylactic agent for psoriasis of the present invention is provided as a pharmaceutical product for parenteral administration, examples of the dosage form thereof include injections, infusions, transdermal formulations, ointments, creams, gels, lotions, sprays, foams, pastes, suppositories, pellets, intranasal formulations, transpulmonary formulations, eye drops, and the like.

The therapeutic or prophylactic agent for psoriasis of the present invention may be used in combination with other drugs for the treatment or prevention of psoriasis (e.g., vitamin D3 derivatives, vitamin D2 derivatives, vitamin E, retinoids, steroids, immunosuppressive drugs, immunomodulatory drugs, nonsteroidal anti-inflammatory drugs, cyclooxygenase inhibitors, PDE4 inhibitors, TNF-α inhibitors, antihistamines, inhibitors of molecules involved in signal transduction, interleukin inhibitors, interleukin receptor antagonists, interleukins, MAPK inhibitors, tyrosine kinase inhibitors, cytokine production inhibitors, JAK inhibitors, T cell inhibitors, B cell inhibitors, antimetabolites, gold preparations, co-stimulatory molecule-related proteins, and dihydroorotate dehydrogenase (DHODH) inhibitors) (which are hereinafter referred to as combinable drugs).

The serotonin 4 receptor agonist (active ingredient in the present invention) and any of the combinable drugs may be formulated simultaneously and administered as a single preparation, or the serotonin 4 receptor agonist and any of the combinable drugs may be formulated separately and administered to the same subject simultaneously or separately with a time lag, by the same or different routes.

The therapeutic or prophylactic agent for psoriasis of the present invention may be a pharmaceutical composition containing a serotonin 4 receptor agonist and at least one or more pharmaceutically acceptable carriers.

The therapeutic or prophylactic agent for psoriasis of the present invention can be produced, for example, by mixing a serotonin 4 receptor agonist and at least one pharmaceutically acceptable carrier in accordance with methods known in the field of pharmaceutical formulations.

Examples of the pharmaceutically acceptable carrier include: excipients, disintegrants, binders, fluidizers, lubricants, and the like in solid preparations; solvents, dissolution aids, suspending agents, isotonic agents, buffers, pH adjusters, analgesic agents, and the like in liquid preparations; and bases, emulsifiers, wetting agents, stabilizers, stabilizing agents, dispersing agents, plasticizers, pH adjusters, absorption promoters, gelling agents, viscosity agents, antiseptics, fillers, dissolving agents, dissolution aids, suspending agent, and the like in semisolid preparations. In addition, additives such as preservatives, antioxidants, colorants, and sweetening agents may be used as needed.

In the therapeutic or prophylactic agent for psoriasis of the present invention, the content of serotonin 4 receptor agonist varies depends on the dosage form, type of serotonin 4 receptor agonist, dosage amount, and the like, and can be selected as appropriate.

In the therapeutic or prophylactic agent for psoriasis of the present invention, the content of serotonin 4 receptor agonist may be, for example, from 0.1% by weight to 100% by weight of the total formulation.

The therapeutic or prophylactic agent for psoriasis of the present invention is effective for the treatment or prevention of psoriasis in a subject of administration.

In the present invention, "psoriasis" includes psoriasis vulgaris, psoriatic arthritis, guttate psoriasis, psoriatic erythroderma, and pustular psoriasis.

In the present invention, "treatment" means the act of administering the agent of the present invention to an individual who has developed psoriasis, and also includes the act of administering the agent of the present invention to improve psoriasis, prevent or delay aggravation, maintain remission, prevent flare-up, or prevent relapse. In the present invention, "prevention" means the act of administering the agent of the present invention to an individual who has not developed psoriasis, and also includes the act of administering the agent of the present invention to prevent or delay the onset of psoriasis.

The therapeutic or prophylactic agent for psoriasis of the present invention can be safely administered to humans and non-human mammals (e.g., mice, rats, hamsters, rabbits, cats, dogs, cows, horses, pigs, sheep, and monkeys).

The dosage of the therapeutic or prophylactic agent for psoriasis of the present invention varies depending on a subject of administration, symptoms, dosage form, route of administration, and the like. For example, when orally administered to an adult patient (weighing approximately 60 kg), the dosage of the active ingredient, serotonin 4 receptor agonist, per day is usually from 0.01 mg to 500 mg, preferably from 0.1 mg to 50 mg, more preferably from 1 mg to 30 mg, once a day or divided into several times a day.

Hereinafter, the present invention will be more specifically described by way of examples and test examples, but the present invention is not limited to these examples and test examples.

### Examples

The compounds used in Test Examples 1 to 5 below and their abbreviations are listed in Table 1. The compounds used in Test Examples 6 and 7 below and their abbreviations are listed in Table 2. Serotonin is abbreviated as 5-HT.

**[Table 1]**

| Type | Compound | Abbreviation | Manufacturer or provider |
|---|---|---|---|
| 5-HT4 receptor agonist | Mosapride citrate dihydrate | MSP | eNovation Chemicals |
| | Prucalopride succinate | PCP | MedChemExpress |
| | Velusetrag | VRG | Axon Medchem |
| | Cinitapride tartrate | CNP | Toronto Research Chemicals |
| | Clebopride malate | CBP | Haoyuan Chemexpress |
| | Tegaserod maleate | TGR | MedChemExpress |
| | Cisapride | CSP | MedChemExpress |
| Positive control | Apremilast | APR | Carbosynth |
| 5-HT1 receptor agonist | Rizatriptan benzoate | RZT | Angene |
| | Tandospirone citrate | TDS | BLD Pharmatech |
| 5-HT2 receptor agonist | Lorcaserin hydrochloride | LCS | Biochempartner |

**[Table 2]**

| Type | Compound | Abbreviation | Manufacturer or provider |
|---|---|---|---|
| | Mosapride citrate dihydrate | MSP | eNovation Chemicals |
| | Felcisetrag | FCT | ChemScene |
| | Usmarapride | UMP | MedChemExpress |
| | Relenopride hydrochloride | RLP | ChemScene |
| 5-HT4 receptor agonist | Renzapride hydrochloride dihydrate | RZP | Wuxi App Tec |
| | Naronapride hydrochloride | NRP | Wuxi App Tec |
| | CJ-033466 | - | ChemExpress |
| | Revexepride | RVP | MedChemExpress |
| | E-3620 | - | ChemExpress |
| Positive control | Apremilast | APR | Carbosynth |

[Test Example 1] Effect of 5-HT4 Receptor Agonist (Comparison with 5-HT1 Receptor Agonist and 5-HT2 Receptor Agonist)

The following test was conducted in order to confirm the effect of 5-HT receptor 4 agonist on dermatitis in psoriasis model mice.

15 mg/ear of imiquimod cream (product name: Beselna cream, manufactured by Mochida Pharmaceutical Co., Ltd.) was repeatedly applied to the right auricle of male BALB/cCrSlc mice (9 weeks old) at once a day for 4 days (Day 0 to Day 3) to prepare psoriasis model mice. One hour before the imiquimod application, Vehicle (0.5% methylcellulose (hereinafter abbreviated as 0.5% MC)), 3 mg/kg body weight MSP (5-HT4 receptor agonist), 3 mg/kg body weight RZT (5-HT1 receptor agonist), 3 mg/kg body weight TDS (5-HT1 receptor agonist), 3 mg/kg body weight LCS (5-HT2 receptor agonist), and 10 mg/kg body weight APR (positive control) were orally administered at 10 mL/kg body weight each (Day 0 to Day 3) (n=6 per administration group). The auricular thickness was measured before the imiquimod cream application (Day 0) and approximately 24 hours after the final administration of each evaluation substance (Day 1 to Day 4), and the difference in auricular thickness (auricular thickening) between Day 0 and Day 4 was evaluated as the degree of inflammation. Day 0 was defined as the day on which the imiquimod cream application was started.

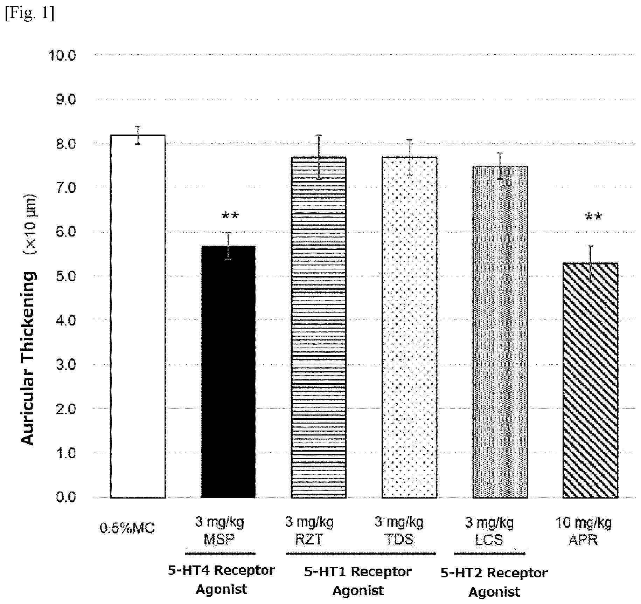

FIG. 1 shows the results (auricular thickening (mean value) of each administration group). In the diagram, ** indicates a significant difference against the 0.5% MC group (** P<0.01). When compared with 0.5% MC, 3 mg/kg body weight MSP (5-HT4 receptor agonist) (example of the present invention) significantly suppressed the auricular thickening as with 10 mg/kg body weight APR (positive control). On the other hand, none of 3 mg/kg body weight RZT (5-HT1 receptor agonist) (comparative example), 3 mg/kg body weight TDS (5-HT1 receptor agonist) (comparative example), and 3 mg/kg body weight LCS (5-HT2 receptor agonist) (comparative example) suppressed the auricular thickening.

### [Test Example 2] Dose-Response of MSP (5-HT4 Receptor Agonist)

The following test was conducted in order to confirm the effect (dose-response) of MSP (5-HT receptor 4 agonist) on dermatitis in psoriasis model mice.

Psoriasis model mice were prepared by the same method as in Test Example 1, and Vehicle (0.5% MC), 3 mg/kg body weight MSP, 30 mg/kg body weight MSP, and 10 mg/kg body weight APR (positive control) were orally administered at 10 mL/kg body weight each (Day 0 to Day 3) (n=6 per administration group). The difference in auricular thickness (auricular thickening) between Day 0 and Day 4 was evaluated as the degree of inflammation.

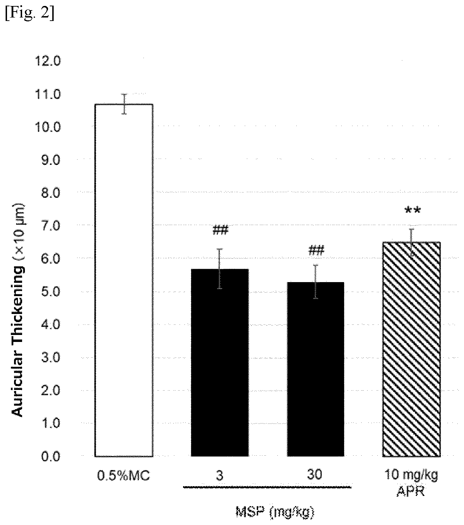

FIG. 2 shows the results (auricular thickening (mean value) of each administration group). In the diagram, ** indicates a significant difference when a two-group test was conducted for the 0.5% MC group (** P<0.01), and ## indicates a significant difference when a multi-group test was conducted for the 0.5% MC group (## P<0.01). When compared with the 0.5% MC, both 3 mg/kg body weight MSP (example of the present invention) and 30 mg/kg body weight MSP (example of the present invention) significantly suppressed the auricular thickening in a dose-dependent manner, suggesting that their suppressing effect was equivalent to that of 10 mg/kg body weight APR (positive control).

### [Test Example 3] Dose-Response of PCP and VRG (5-HT4 Receptor Agonists)

The following test was conducted in order to confirm the effect (dose-response) of PCP and VRG (5-HT receptor 4 agonists) on dermatitis in psoriasis model mice. MSP (5-HT receptor 4 agonist), whose dose-response was confirmed in Test Example 2 above, was also tested.

Psoriasis model mice were prepared by the same method as in Test Example 1, and Vehicle (0.5% MC), 3 mg/kg body weight MSP, 3 mg/kg body weight PCP, 10 mg/kg body weight PCP, 30 mg/kg body weight PCP, 3 mg/kg body weight VRG, 10 mg/kg body weight VRG, 30 mg/kg body weight VRG, and 10 mg/kg body weight APR (positive control) were orally administered at 10 mL/kg each (Day 0 to Day 3) (n=6 per administration group). The difference in auricular thickness (auricular thickening) between Day 0 and Day 4 was evaluated as the degree of inflammation.

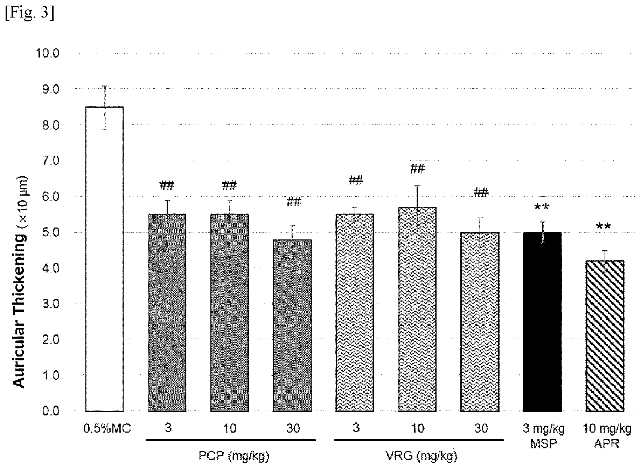

FIG. 3 shows the results (auricular thickening (mean value) of each administration group). In the diagram, ** indicates a significant difference when a two-group test was conducted for the 0.5% MC group (** P<0.01), and ## indicates a significant difference when a multi-group test was conducted for the 0.5% MC group (## P<0.01). When compared with the 0.5% MC, PCP (example of the present invention) and VRG (example of the present invention) significantly suppressed the auricular thickening at all doses as with 10 mg/kg body weight APR (positive control) and 3 mg/kg body weight MSP (example of the present invention).

### [Test Example 4] Effect of 5-HT4 Receptor Agonists (CNP and CBP)

The following test was conducted in order to confirm the effect of CNP and CBP (5-HT receptor 4 agonists) on dermatitis in psoriasis model mice. MSP and VRG (5-HT receptor 4 agonists), whose dose-response was confirmed in Test Examples 2 and 3 above, were also tested.

Psoriasis model mice were prepared by the same method as in Test Example 1, and Vehicle (0.5% MC), 30 mg/kg body weight MSP, 30 mg/kg body weight CNP, 30 mg/kg body weight VRG, 30 mg/kg body weight CBP, and 10 mg/kg body weight APR (positive control) were orally administered at 10 mL/kg body weight each (Day 0 to Day 3) (n=6 per administration group). The difference in auricular thickness (auricular thickening) between Day 0 and Day 4 was evaluated as the degree of inflammation.

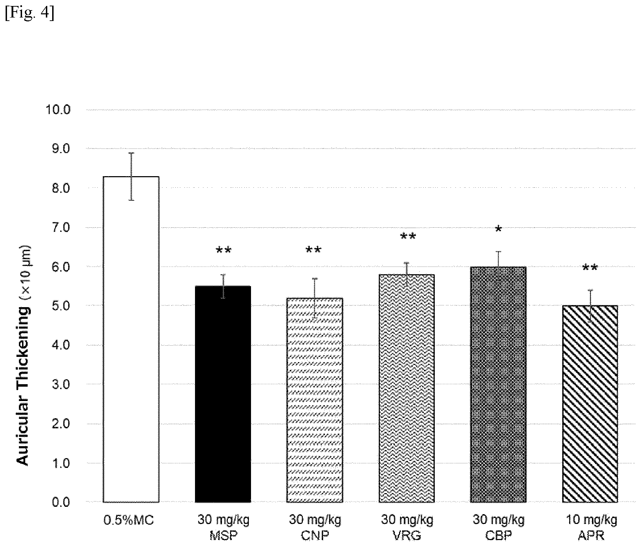

FIG. 4 shows the results (auricular thickening (mean value) of each administration group). In the diagram, * and ** indicate a significant difference against the 0.5% MC group (* P<0.05, ** P<0.01). When compared with the 0.5% MC, 30 mg/kg body weight MSP (example of the present invention), 30 mg/kg body weight CNP (example of the present invention), 30 mg/kg body weight VRG (example of the present invention), and 30 mg/kg body weight CBP (example of the present invention) all significantly suppressed the auricular thickening as with 10 mg/kg body weight APR (positive control).

### [Test Example 5] Effect of 5-HT4 Receptor Agonists (CSP and TGR)

The following test was conducted in order to confirm the effect of CSP and TGR (5-HT receptor 4 agonists) on dermatitis in psoriasis model mice. PCP (5-HT receptor 4 agonist), whose dose-response was confirmed in Test Example 3 above, was also tested.

Psoriasis model mice were prepared by the same method as in Test Example 1, and Vehicle (0.5% MC), 30 mg/kg body weight PCP, 30 mg/kg body weight CSP, 30 mg/kg body weight TGR, and 10 mg/kg body weight APR (positive control) were orally administered at 10 mL/kg body weight each (Day 0 to Day 3) (n=6 per administration group). The difference in auricular thickness (auricular thickening) between Day 0 and Day 4 was evaluated as the degree of inflammation.

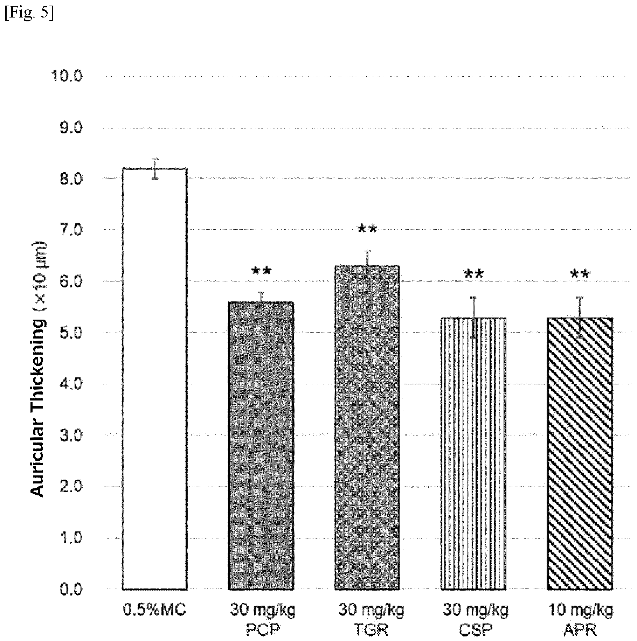

FIG. 5 shows the results (auricular thickening (mean value) of each administration group). In the diagram, ** indicates a significant difference against the 0.5% MC group (** P<0.01). When compared with the 0.5% MC, 30 mg/kg body weight PCP (example of the present invention), 30 mg/kg body weight CSP (example of the present invention), and 30 mg/kg body weight TGR (example of the present invention) all significantly suppressed the auricular thickening as with 10 mg/kg body weight APR (positive control).

### [Test Example 6] Effect of 5-HT4 Receptor Agonists (FCT, UMP, RLP, and RZP)

The following test was conducted in order to confirm the effect of FCT, UMP, RLP, and RZP (5-HT receptor 4 agonists) on dermatitis in psoriasis model mice.

Psoriasis model mice were prepared by the same method as in Test Example 1, and Vehicle (0.5% MC), 30 mg/kg body weight FCT, 30 mg/kg body weight UMP, 30 mg/kg body weight RLP, 30 mg/kg body weight RZP, 30 mg/kg body weight MSP, and 10 mg/kg body weight APR (positive control) were orally administered at 10 mL/kg body weight each (Day 0 to Day 3) (n=6 per administration group). The difference in auricular thickness (auricular thickening) between Day 0 and Day 4 was evaluated as the degree of inflammation.

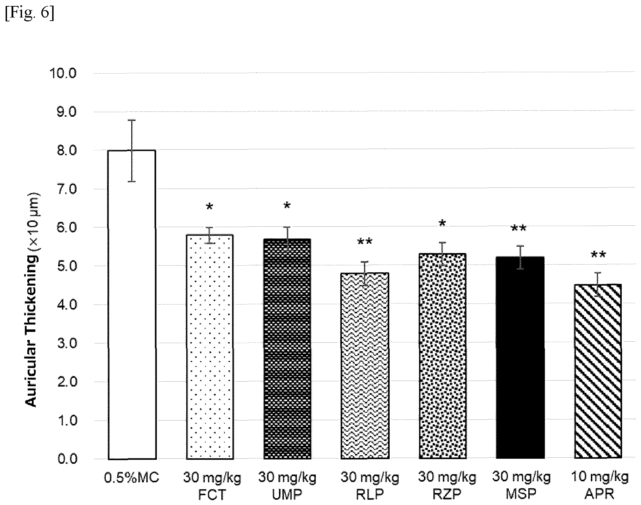

FIG. 6 shows the results (auricular thickening (mean value) of each administration group). In the diagram, * and ** indicate a significant difference when a two-group test was conducted for the 0.5% MC group (* P<0.05, ** P<0.01). When compared with the 0.5% MC, 30 mg/kg body weight FCT (example of the present invention), 30 mg/kg body weight UMP (example of the present invention), 30 mg/kg body weight RLP (example of the present invention), and 30 mg/kg body weight RZP (example of the present invention) all significantly suppressed the auricular thickening as with 10 mg/kg body weight APR (positive control) and 30 mg/kg body weight MSP (example of the present invention).

### [Test Example 7] Effect of 5-HT4 receptor agonists (NRP, CJ-033466, RVP, and E-3620)

The following test was conducted in order to confirm the effect of NRP, CJ-033466, RVP, and E-3620 (5-HT receptor 4 agonists) on dermatitis in psoriasis model mice.

Psoriasis model mice were prepared by the same method as in Test Example 1, and Vehicle (0.5% MC), 30 mg/kg body weight NRP, 30 mg/kg body weight CJ-033466, 30 mg/kg body weight RVP, 30 mg/kg body weight E-3620, 30 mg/kg body weight MSP, and 10 mg/kg body weight APR (positive control) were orally administered at 10 mL/kg body weight each (Day 0 to Day 3) (n=6 per administration group). The difference in auricular thickness (auricular thickening) between Day 0 and Day 4 was evaluated as the degree of inflammation.

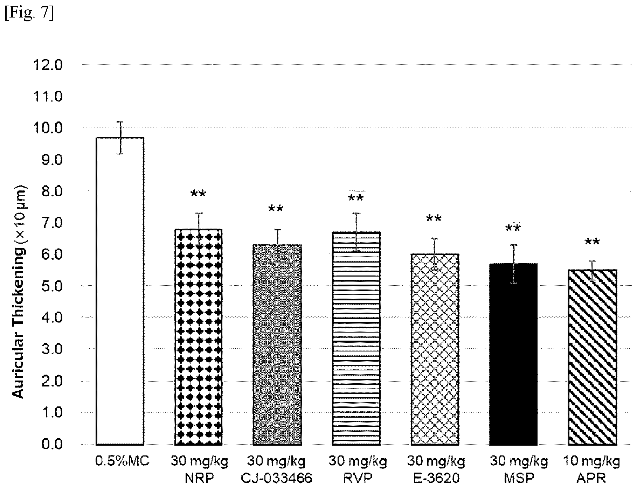

FIG. 7 shows the results (auricular thickening (mean value) of each administration group). In the diagram, ** indicates a significant difference when a two-group test was conducted for the 0.5% MC group (** P<0.01). When compared with the 0.5% MC, 30 mg/kg body weight NRP (example of the present invention), 30 mg/kg body weight CJ-033466 (example of the present invention), 30 mg/kg body weight RVP (example of the present invention), and 30 mg/kg body weight E-3620 (example of the present invention) all significantly suppressed the auricular thickening as with 10 mg/kg body weight APR (positive control) and 30 mg/kg body weight MSP (example of the present invention).

The results of Test Examples 1 to 7 above suggest that 5-HT4 receptor agonists are effective in the treatment of psoriasis.

### Industrial Applicability

The present invention makes it possible to provide a novel therapeutic or prophylactic agent for psoriasis.

The present application claims the benefit of priority based on Japanese Patent Application No. 2022-106025, which is incorporated herein by reference in its entirety.

## Claims

1. A therapeutic or prophylactic agent for psoriasis, comprising a serotonin 4 receptor agonist.

2. The agent according to claim 1,
wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.

3. The agent according to claim 1 or 2, which is for oral administration.

4. A method for treating or preventing psoriasis, comprising a step of administering an effective amount of serotonin 4 receptor agonist to a subject.

5. The method according to claim 4,
wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.

6. The method according to claim 4 or 5,
wherein the administration is oral administration.

7. A serotonin 4 receptor agonist for use in treatment or prevention of psoriasis.

8. The serotonin 4 receptor agonist according to claim 7,
wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.

9. The serotonin 4 receptor agonist according to claim 7 or 8, for use in oral administration.

10. Use of a serotonin 4 receptor agonist in the manufacture of a therapeutic or prophylactic agent for psoriasis.

11. The use according to claim 10,
wherein the serotonin 4 receptor agonist is one or at least two selected from the group consisting of mosapride, prucalopride, velusetrag, tegaserod, cisapride, cinitapride, clebopride, felcisetrag, naronapride, usmarapride, relenopride, renzapride, CJ-033466, revexepride, E-3620, and pharmaceutically acceptable salts thereof.

12. The use according to claim 10 or 11,
wherein the therapeutic or prophylactic agent for psoriasis is for oral administration.
